# EUROPÄISCHE PATENTSCHRIFT

(11) **EP 2 542 204 B1**
(45) Veröffentlichungstag und Bekanntmachung des Hinweises auf die Patenterteilung: **16.07.2014**
(21) Anmeldenummer: 11704575.7
(22) Anmeldetag: 08.02.2011
(51) Int. Cl.: A61J 1/16, A61M 5/00, A61M 5/14

(54) **FLASCHENHALTER FÜR EINE INJEKTIONSVORRICHTUNG**
BOTTLE HOLDER FOR AN INJECTION DEVICE
PORTE-FLACON POUR UN DISPOSITIF D'INJECTION

(30) Priorität: 01.03.2010 DE 102010000593
(43) Veröffentlichungstag der Anmeldung: 09.01.2013
(73) Patentinhaber: Ulrich GmbH & Co. KG, 89081 Ulm (DE)
(72) Erfinder: KAISER, Jörg, 79312 Emmendingen (DE); KRANHOLD, Thomas, 06846 Dessau (DE)
(74) Vertreter: Charrier, Rapp & Liebau
(86) Internationale Anmeldenummer: PCT/EP2011/051816
(87) Internationale Veröffentlichungsnummer: WO 2011/107327

(56) Entgegenhaltungen:
- EP-A1- 2 011 540
- EP-A2- 1 219 283
- US-A- 6 070 761

## Beschreibung

Die Erfindung betrifft einen Flaschenhalter für eine Injektionsvorrichtung nach dem Oberbegriff des Anspruchs 1.

Injektionsvorrichtungen werden im Bereich der Medizintechnik zur Injektion von Flüssigkeiten in den Körper eines Patienten verwendet. Solche Injektionsvorrichtungen können beispielsweise zur Verabreichung von Kontrastmitteln bei der Durchführung von bildgebenden Verfahren wie Computertomographie, Ultraschalluntersuchungen und Magnetresonanztomographie (MRT) verwendet werden. Die zu injizierenden Flüssigkeiten, wie z.B. verschiedene Kontrastmittel und NaCl-Spüllösungen, sind dabei in Vorratsflaschen abgefüllt. Die Flaschen mit den zu injizierenden Flüssigkeiten werden bspw. am oberen Ende eines in der Regel rollengelagerten Ständers aufgehängt und über einen Zufuhrschlauch zur Injektionsvorrichtung geführt. Die Injektionsvorrichtung umfasst eine Pumpe, beispielsweise eine Schlauchpumpe, mit welcher die in den Zufuhrschläuchen geführten Flüssigkeiten in einen intravenös mit dem Patienten verbundenen Patientenschlauch gepumpt werden. Diese Anordnung erweist sich als nachteilig, weil beim Anschließen einer neuen Vorratsflasche der Zufuhrschlauch zwischen der Vorratsflasche und der Pumpe der Injektionsvorrichtung entlüftet werden muss. Dabei kann Flüssigkeit austreten, die zu Verschmutzungen führen kann. Zudem weisen die Ständer, an denen die Vorratsflaschen aufgehängt werden, häufig nicht die erforderliche Standfestigkeit auf und können unbeabsichtigt umgestoßen werden.

Zur Beseitigung dieser Nachteile wird in der Gebrauchsmusterschrift DE 203 06 395 U1 ein Kontrastmittelgerät vorgeschlagen, welches wenigstens eine Flaschenaufnahme zur Aufnahme einer Kontrastmittelflasche sowie eine Dosimeterpumpe zur Dosierung des Kontrastmittels aufweist, wobei das Kontrastmittel aus einer in die Flaschenaufnahme gestellten Kontrastmittelflasche mittels der Dosimeterpumpe an einen an einer Braunüle anschließbaren Patientenschlauch gefördert werden kann. Am Boden der oder jeder Flaschenaufnahme ist ein hohler Dom zum Anstechen eines durchstechbaren Verschlusses der Kontrastmittelflasche vorgesehen. Beim Einstecken einer Kontrastmittelflasche in die Flaschenaufnahme wird die Kontrastmittelflasche durch den Anstechdorn geöffnet, so dass das Kontrastmittel aus der Kontrastmittelflasche in den hohlen Anstechdorn und über eine sich daran anschließende Leitung zunächst in einen Tank fließen kann. Der Tank steht über Verbindungsleitungen mit der Dosimeterpumpe in Verbindung, so dass die Dosimeterpumpe Kontrastmittel aus dem Tank ansaugen und zu dem intravenös mit dem Patienten verbundenen Patientenschlauch fördern kann. Die oder jede Flaschenaufnahme ist bei diesem Kontrastmittelgerät durch einen becherförmigen Flaschenhalter ausgebildet, der im Bodenbereich eine Ausnehmung zur Aufnahme des Flaschenkopfes und des Flaschenhalses der Kontrastmittelflasche aufweist. Im Zentrum dieser Ausnehmung ist der hohle Anstechdom angeordnet. Zur Befüllung des Kontrastmittelgeräts mit einer gefüllten Vorratsflasche muss der Bediener die Vorratsflasche kopfüber (also mit dem Flaschenhals nach unten weisend) in die Flaschenaufnahme einstecken und auf den Anstechdom drücken, bis der Anstechdom eine Auslauföffnung in die Kontrastmittelflasche eingestochen hat.

Dieses Vorgehen ist umständlich und zeitaufwendig und erfordert einiges Geschick vom Bedienpersonal. Insbesondere besteht die Gefahr, dass der Bediener die Kontrastmittelflasche nicht zentriert auf den Anstechdorn aufsteckt, mit der Folge, dass der Anstechdom keine Auslauföffnung durch den Verschlussdeckel der Kontrastmittelflasche einstechen kann. Dieses Problem tritt insbesondere bei kleineren Kontrastmittelflaschen auf, deren Durchmesser kleiner ist als der Innendurchmesser der Flaschenaufnahme, weil in diesem Falle die Innenwandung der becherförmigen Flaschenaufnahme keine Führung der Kontrastmittelflasche beim Aufstecken auf den Anstechdom gewährleisten kann. Die bekannte Anordnung erweist sich darüber hinaus als nachteilig, weil auch keine größeren Kontrastmittelflaschen verwendet werden können, deren Durchmesser größer ist als der Innendurchmesser der Flaschenaufnahme.

Ein weiterer Nachteil der bekannten Vorrichtung ergibt sich beim Abziehen einer geleerten Kontrastmittelflasche aus der Flaschenaufnahme. Die Flasche wird nämlich in vertikaler Richtung nach oben aus der Flaschenaufnahme herausgezogen, wobei Restflüssigkeit aus der Auslassöffnung der Kontrastmittelflasche austropfen kann. Austropfende Restflüssigkeit führt wiederum zu Verschmutzungen des Kontrastmittelgeräts.

Aus der US 6,070,761 A ist eine Injektionsvorrichtung mit einem Flaschenhalter bekannt, welche eine Flaschenaufnahme zur Aufnahme einer Vorratsflasche mit einem Arzneimittel und einen am Flaschenhalter befestigten Einstechdorn zum Einstechen einer Auslauföffnung in die Vorratsflasche umfasst, wobei die Flaschenaufnahme bezüglich des Einstechdorns in vertikaler Richtung beweglich ist und bei einer Bewegung der Flaschenaufnahme von ihrer oberen Grenzstellung in ihre untere Grenzstellung eine in der Flaschenaufnahme angeordnete Vorratsflasche so auf den Einstechdorn aufgesetzt wird, dass dieser eine Auslauföffnung in die Vorratsflasche einsticht. Die EP 2 011 540 A1 zeigt einen verschwenkbaren Flaschenhalter in einer Injektionsvorrichtung zur Injektion von Kontrastmitteln. Durch die Verschwenkbarkeit des Flaschenhalters kann verhindert werden, dass beim Wechseln einer geleerten Vorratsflasche Restflüssigkeit aus der Vorratsflasche austritt.

Hiervon ausgehend liegt der Erfindung die Aufgabe zugrunde, einen Flaschenhalter für eine Injektionsvorrichtung aufzuzeigen, mit dem eine möglichst einfache und schnelle Bestückung der Injektionsvorrichtung mit einer gefüllten Vorratsflasche ermöglicht wird, wobei der Flaschenhalter so ausgebildet sein soll, dass er verschiedene Vorratsflaschen unterschiedlicher Größe aufnehmen kann. Der Erfindung liegt ferner die Aufgabe zugrunde, einen Flaschenhalter für eine Injektionsvorrichtung aufzuzeigen, bei dem eine geleerte Vorratsflasche auf möglichst einfache Art und ohne zu tropfen aus dem Flaschenhalter entnommen werden kann.

Diese Aufgaben werden mit einer Injektionsvorrichtung für einen Flaschenhalter mit den Merkmalen des Anspruchs 1 gelöst. Bevorzugte Ausgestaltungen dieses Flaschenhalters sind den abhängigen Ansprüchen 2 bis 15 zu entnehmen.

Der erfindungsgemäße Flaschenhalter weist eine Flaschenaufnahme zur Aufnahme einer Vorratsflasche sowie einen am Flaschenhalter befestigten Einstechdorn zum Einstechen einer Auslassöffnung in die Vorratsflasche oder alternativ eine Halteeinrichtung zum Anbringen eines austauschbaren Einstechdorns auf. Die Flaschenaufnahme ist bezüglich des fest am Flaschenhalter angeordneten Einstechdorns oder bezüglich der Halteeinrichtung für den austauschbaren Einstechdorn verschiebbar. Zur Bestückung einer mit einem solchen Flaschenhalter ausgerüsteten Injektionsvorrichtung mit einer gefüllten Vorratsflasche wird diese kopfüber in die Flaschenaufnahme gesteckt und in vertikaler Richtung nach unten gedrückt. Dadurch bewegt sich die bewegliche Flaschenaufnahme in Richtung des Einstechdorns und setzt die Vorratsflasche so auf den Einstechdorn auf, dass dieser eine Auslauföffnung in der Vorratsflasche einsticht. Durch die geführte Bewegung der Flaschenaufnahme gegenüber dem Einstechdorn ist zu jeder Zeit gewährleistet, dass die Vorratsflasche und insbesondere ihr Flaschenhals zentriert auf dem Einstechdorn aufgesetzt wird, so dass der Einstechdorn eine Auslassöffnung bspw. durch einen Deckel oder eine dünne Membran, welche den Hals der Vorratsflasche verschließt, einstechen kann.

Die Bewegung der Flaschenaufnahme bezüglich des Einstechdorns wird dabei bevorzugt durch eine Führungsanordnung geführt, welche mit einer Feder gekoppelt ist. Die Flaschenaufnahme ist dann entgegen der Rückstellkraft der Feder, welche insbesondere als Druckfeder ausgebildet ist, zwischen einer oberen Grenzstellung und einer unteren Grenzstellung verschiebbar. Zweckmäßig ist die Flaschenaufnahme in der oberen und /oder in der unteren Grenzstellung über einen Rastmechanismus fixierbar. In ihrer oberen Grenzstellung ist die Flaschenaufnahme bereit zur Aufnahme einer neuen, gefüllten Vorratsflasche. Nach Einfügen einer gefüllten Vorratsflasche in die Flaschenaufnahme wird die Flasche in vertikaler Richtung nach unten gedrückt, wodurch sich die Flaschenaufnahme auf den darunter angeordneten Einstechdorn zubewegt, bis die untere Grenzstellung der Flaschenaufnahme erreicht ist. Sobald die Flaschenaufnahme die untere Grenzstellung erreicht hat, hat der Einstechdorn eine Auslassöffnung in die Vorratsflasche eingestochen. Der zweckmäßig hohle Anstechdorn ist mit einer Zufuhrleitung verbunden, über welche dann die Flüssigkeit aus der Vorratsflasche in eine Pumpe der Injektionsvorrichtung geleitet werden kann.

Um Vorratsflaschen unterschiedlicher Form und Größe sicher in die Flaschenaufnahme des erfindungsgemäßen Flaschenhalters stellen zu können, ist bei dem erfindungsgemäßen Flaschenhalter bevorzugt ein trichterförmiges Aufnahmeelement vorgesehen, in welches der Hals einer Vorratsflasche eingeführt und dort gehalten werden kann. Ferner ist bevorzugt eine Halteeinrichtung an der Flaschenaufnahme angeordnet, welche über flexible bzw. biegbare Halteelemente verfügt. Die flexiblen Halteelemente umgreifen die Vorratsflasche zumindest teilweise an ihrem Außenumfang und fixieren diese so in der Flaschenaufnahme. Durch die flexible bzw. biegbare Ausbildung der Halteelemente passen sich diese der Größe und der Form der Vorratsflasche an und liegen kraftschlüssig am Außenumfang der Vorratsflasche an, um diese in der Flaschenaufnahme zu fixieren. So können auch Flaschen unterschiedlicher Größe und insbesondere mit unterschiedlichem Durchmesser in der Flaschenaufnahme" fixiert werden.

Der erfindungsgemäße Flaschenhalter ist bevorzugt zwischen einer vertikalen Grundstellung und einer horizontalen Schwenkstellung verschwenkbar an einem Gehäuseteil der Injektionsvorrichtung angelenkt. Der Verschwenkmechanismus ist dabei zweckmäßig so ausgestaltet, dass der Flaschenhalter in seiner unteren Grenzstellung gegenüber dem Gehäuseteil der Injektionsvorrichtung so verschwenkbar ist, dass die in der Flaschenaufnahme befindliche Vorratsflasche in eine im Wesentlichen horizontale Lage gebracht werden kann. In dieser horizontalen Lage kann die Vorratsflasche aus der Flaschenaufnahme herausgezogen werden, ohne dass Restflüssigkeit aus der Auslassöffnung der Vorratsflasche austropft.

Um ein unbeabsichtigtes Ausschwenken des Flaschenhalters in seine horizontale Schwenkstellung zu verhindern, ist zweckmäßig ein Verriegelungsmechanismus vorgesehen, welcher den Flaschenhalter in seiner vertikalen Stellung verriegelt. Zur Entriegelung umfasst der Flaschenhalter einen Entriegelungshebel, um den Flaschenhalter nach dem Lösen der Verriegelung von seiner vertikalen unteren Grenzstellung in die horizontale Stellung verschwenken zu können.

Um sicherzustellen, dass sich die Flaschenaufnahme beim Zurückschwenken des Flaschenhalters von seiner horizontalen Schwenkstellung in die vertikale Grundstellung in ihrer oberen Grenzstellung befindet, in der die Flaschenaufnahme wieder mit einer neuen Vorratsflasche bestückt werden kann, ist bevorzugt eine Zwangsentriegelung vorgesehen, über welche die Flaschenaufnahme beim Zurückschwenken selbsttätig in ihre obere Grenzstellung gebracht wird.

Weitere Vorteile sowie die Handhabung des erfindungsgemäßen Flaschenhalters und einer mit erfindungsgemäßen Flaschenhaltern ausgestatteten Injektionsvorrichtung ergeben sich aus der folgenden Beschreibung eines bevorzugten Ausführungsbeispiels, welches unter Bezugnahme auf die begleitenden Zeichnungen dargestellt wird. Die Zeichnungen zeigen:
- **Fig. 1:**: Vorderansicht einer Injektionsvorrichtung mit drei erfindungsgemäßen Flaschenhalter;
- **Fig.1a:**: Detaildarstellung eines der drei erfindungsgemäßen Flaschenhalter der Injektionsvorrichtung von Figur 1a ;
- **Fig. 2:**: Seitenansicht der Injektionsvorrichtung von Figur 1;
- **Fig. 3a:**: Schnittdarstellung des linken Flaschenhalters von Fig. 1 entlang der Ebene C-C in einer vertikalen Grundstellung;
- **Fig. 3b:**: Schnittdarstellung des rechten Flaschenhalters von Fig. 1 entlang der Ebene B-B in einer vertikalen Grundstellung;
- **Fig. 3c:**: Schnittdarstellung des rechten Flaschenhalters von Fig. 1 entlang der Ebene A-A in einer vertikalen Grundstellung;
- **Fig. 4:**: Perspektivische Darstellung des Flaschenhalters von Fig. 1 in einer Ansicht von hinten;
- **Fig. 5:**: Schnittdarstellungen des Flaschenhalter von Fig. 1a in verschiedenen Funktionsstellungen, wobei Fig. 5a den Flaschenhalter in einer unteren vertikalen Grenzstellung und Fig. 5c den Flaschenhalter in einer ausgeschwenkten Schwenkstellung sowie die Fig. 5b und 5d den Flaschenhalter in Zwischenstellungen zeigen;
- **Fig. 6:**: Detaildarstellung des Flaschenhalters von Fig. 1a in der Zwischenstellung der Figur 5d.

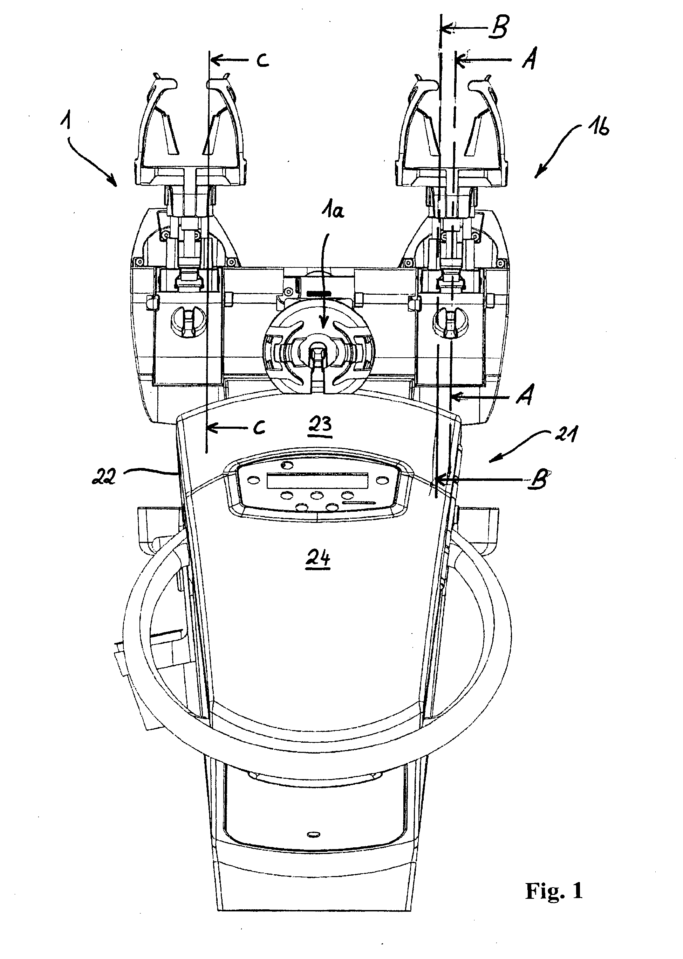

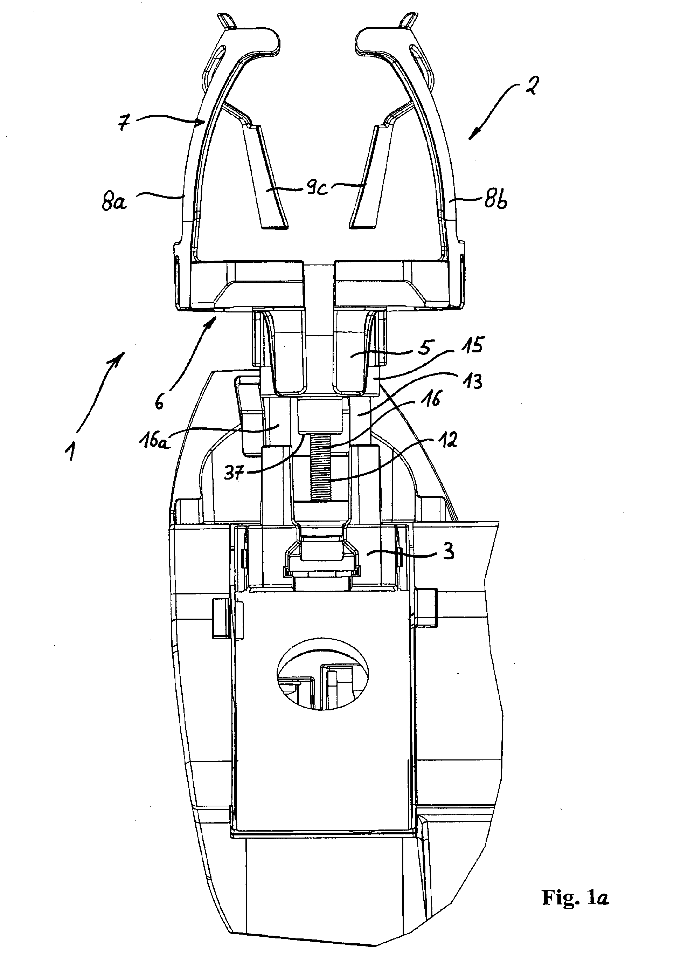

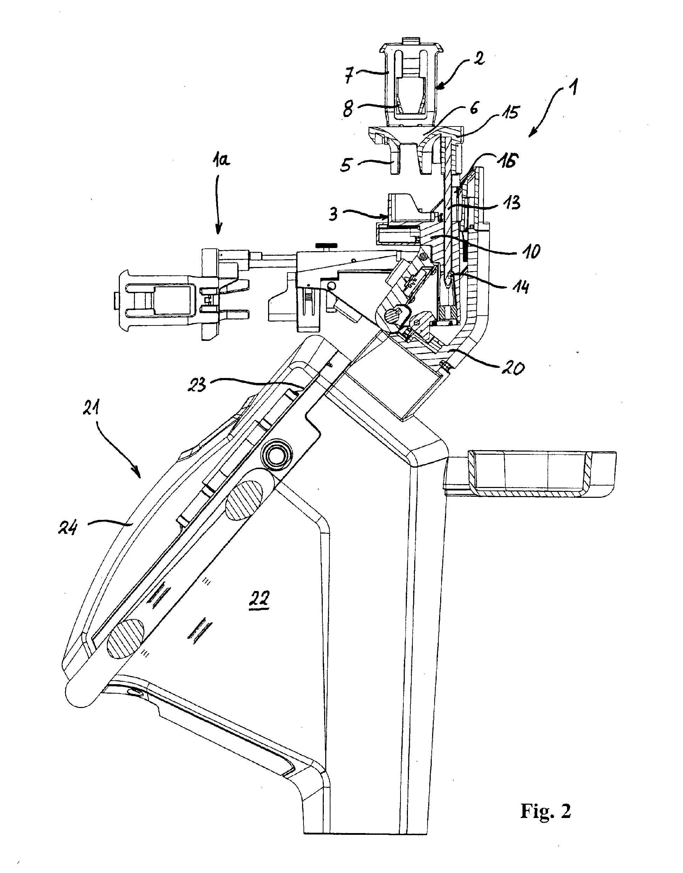

In Fig. 1 ist der Injektionskopf 21 einer Injektionsvorrichtung zur Injektion von zwei verschiedenen oder gleichen Kontrastmitteln und einer NaCl-Spüllösung in den Körper eines Patienten gezeigt, wobei die Injektionsvorrichtung über erfindungsgemäße Flaschenhalter zur Aufnahme von insgesamt drei Vorratsflaschen für die beiden Kontrastmittel und die NaCl-Spüllösung aufweist. Solche Injektionsvorrichtungen werden beispielsweise zur Injektion von Kontrastmitteln bei der Durchführung von bildgebenden Verfahren, wie Computertomographie, Ultraschalluntersuchungen und Magnetresonanztomographie (MRT) verwendet. Der in Fig. 2 gezeigte Injektionskopf 21 umfasst ein Außengehäuse 22, in dem eine Pumpe, bevorzugt eine Schlauchpumpe, zur Förderung der Kontrastmittel und der Spüllösung in einen mit dem Patienten verbundenen Patientenschlauch angeordnet ist. In die Schlauchpumpe wird ein Schlauch eingefügt, in dem die zu injizierenden Flüssigkeiten geführt werden. Die zu injizierenden Flüssigkeiten sind in (hier nicht gezeigten) Vorratsflaschen abgefüllt. Zur Aufnahme der Vorratsflaschen weist der in Fig. 1 gezeigte Injektionskopf 21 insgesamt drei Flaschenhalter 1, 1a, 1b auf. Die drei Flaschenhalter 1, 1a, 1b sind oberhalb eines Panels 23 des Injektionskopfs 21 angeordnet. Das Panel 23 ist mit einem Deckel 24 verschließbar. In der in Fig. 2 gezeigten Seitenansicht ist der Deckel 24 in der geschlossenen Stellung gezeigt. In der Ansicht von Fig. 1 ist der obere Bereich des Panels 23 durch den zweckmäßig transparent ausgebildeten Deckel 24 zu sehen. Im oberen Bereich des Panels 23 sind (hier durch den Deckel 24 abgedeckte und daher nicht sichtbare) kanalförmige Ausnehmungen vorgesehen, in welche eine verzweigte Schlauchanordnung (welche hier zeichnerisch nicht dargestellt ist) eingefügt werden kann. Bei der Schlauchanordnung kann es sich insbesondere um eine Schlauchanordnung handeln, wie sie in der EP 2 011 541 A2 im Detail beschrieben ist. Diese Schlauchanordnung umfasst insgesamt drei Zufuhrschläuche, nämlich einen ersten Zufuhrschlauch für ein erstes Kontrastmittel, einen zweiten Zufuhrschlauch für ein zweites Kontrastmittel und einen dritten Zufuhrschlauch für eine Spüllösung (insbesondere NaCl). Die drei Zufuhrschläuche werden an die Vorratsflaschen für die Kontrastmittel und die Spüllösung angeschlossen und in die im oberen Bereich des Panels 23 angeordneten Verzweigungskanäle eingeklipst. Die von den Vorratsflaschen kommenden Zufuhrschläuche laufen in einem Verzweigungsstück zusammen, welches in einer kreisförmigen Ausnehmung eingefügt wird. Das Verzweigungsstück umfasst einen Ausgangsschlauch, der in die unterhalb des Deckels 24 im Gehäuse 22 angeordnete Pumpe geführt wird.

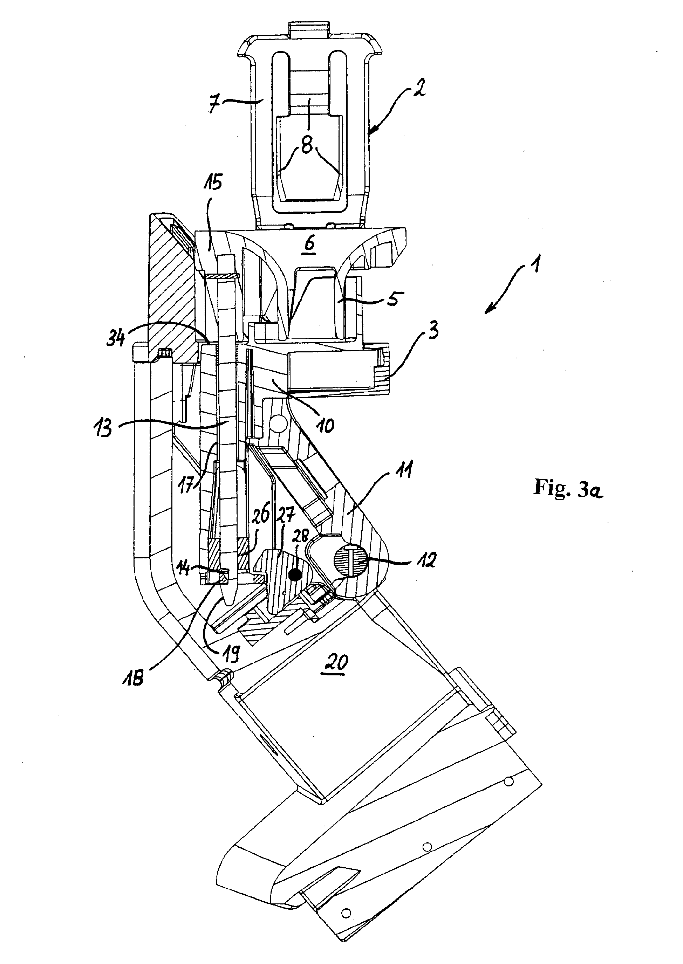

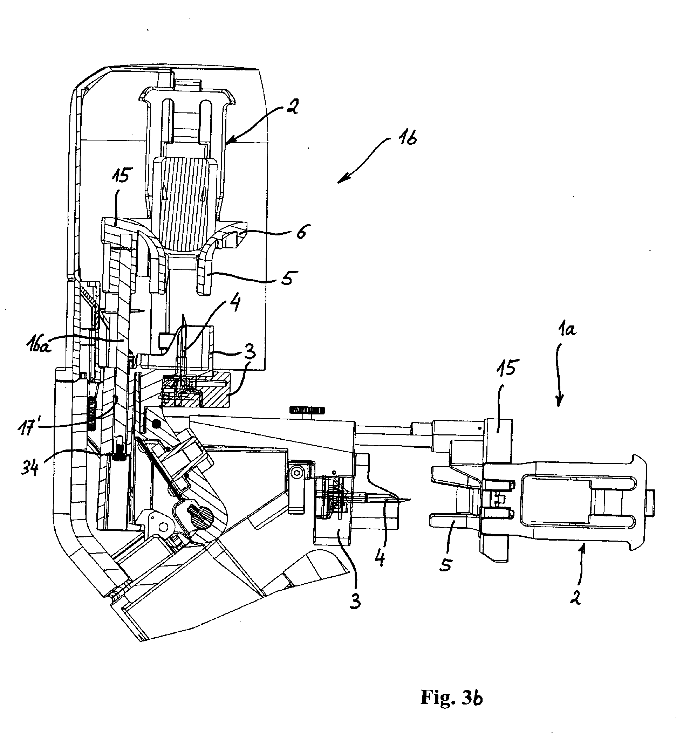

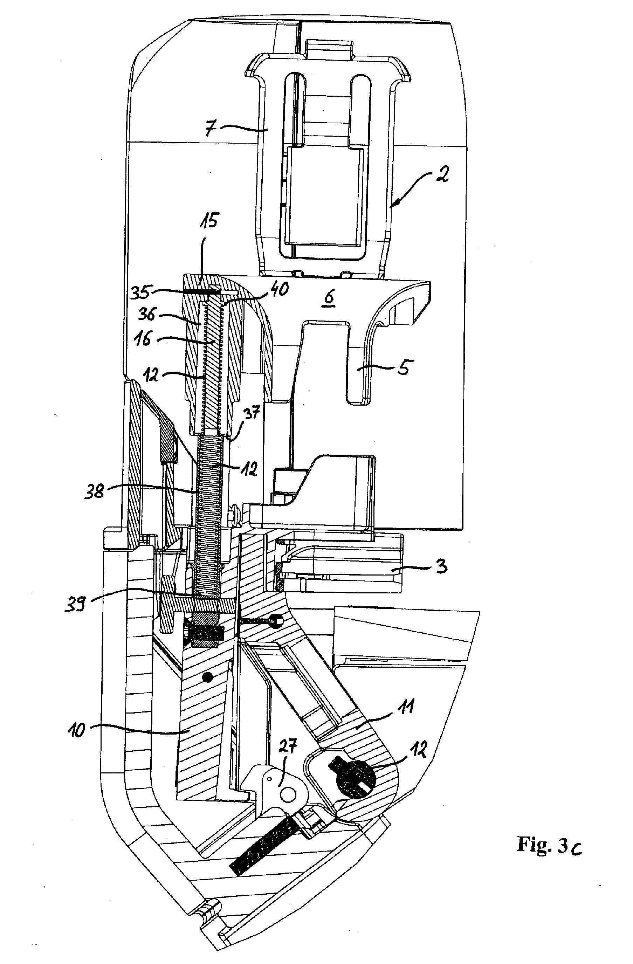

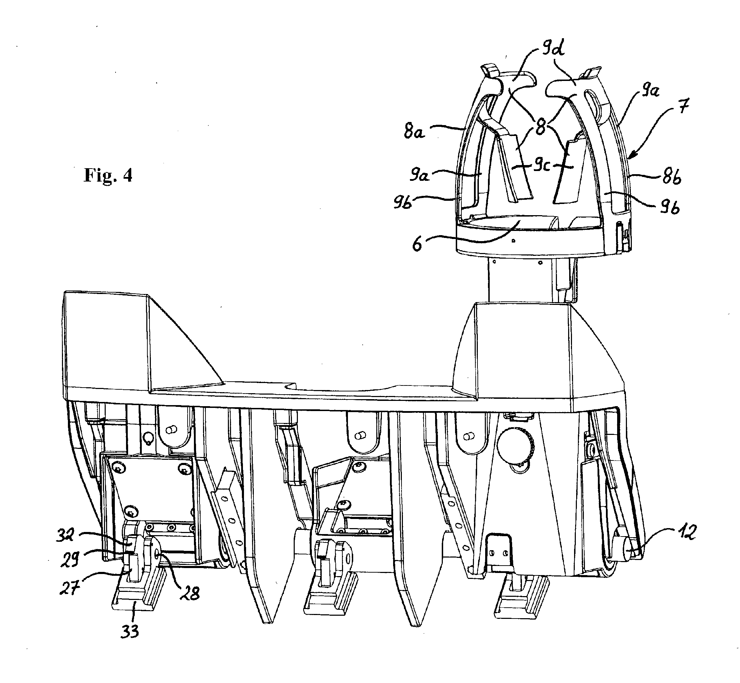

Zur Aufnahme der Vorratsflaschen sind die drei in Fig. 1 gezeigten Flaschenhalter 1, 1a und 1b vorgesehen. Die Flaschenhalter sind jeweils identisch zueinander aufgebaut. Die Einzelheiten des Aufbaus der Flaschenhalter sind aus der Detailzeichnung der Figur 1a sowie den Schnittzeichnungen der Fig. 3 und der perspektivischen Rückansicht der Fig. 4 im Einzelnen zu entnehmen. Die drei Flaschenhalter 1, 1a, 1b sind jeweils verschwenkbar an einem Gehäuseteil 20 des Injektionskopf 21 angelenkt. Jeder der drei Flaschenhalter 1, 1a und 1b ist dadurch zwischen einer vertikalen Grundstellung und einer horizontalen Stellung verschwenkbar. In der Darstellung von Fig. 1 befindet sich der links angeordnete Flaschenhalter 1 und der rechts angeordnete Flaschenhalter 1b jeweils in seiner vertikalen Grundstellung und der mittlere Flaschenhalter 1a ist in seiner horizontalen Stellung. In der vertikalen Stellung kann eine Vorratsflasche in den Flaschenhalter eingeführt und mit einem Zufuhrschlauch verbunden werden. Nach dem Verbinden der in den Flaschenhalter eingestellten Vorratsflasche mit einem Zufuhrschlauch bleibt der Flaschenhalter zur Entnahme der sich in der Vorratsflasche befindlichen Flüssigkeit bei laufender Pumpe in seiner vertikalen Stellung. Ist die Vorratsflasche durch die Förderung der Pumpe geleert worden, kann der Flaschenhalter in seine horizontale Stellung verschwenkt werden. In dieser horizontalen Stellung kann die geleerte Vorratsflasche in horizontaler Richtung aus dem Flaschenhalter herausgezogen werden. Das Herausziehen einer geleerten Vorratsflasche aus dem Flaschenhalter in horizontaler Stellung stellt ein tropffreies Abziehen der geleerten Vorratsflasche vom daran befestigten Zufuhrschlauch sicher. Nach dem Herausziehen der geleerten Vorratsflasche aus dem Flaschenhalter kann dieser wieder in seine vertikale Grundstellung zurückgeschwenkt werden. In der Grundstellung kann dann wieder eine volle Vorratsflasche in vertikaler Richtung von oben in den Flaschenhalter eingefügt werden.

Zur Aufnahme einer Vorratsflasche verfügt jeder der Flaschenhalter 1 über eine Flaschenaufnahme 2 (Figur 1a). Die Flaschenaufnahme 2 umfasst ein trichterförmiges Aufnahmeelement 6, in welches der Flaschenhals einer Vorratsflasche kopfüber eingesteckt werden kann (Figur 2). Die Form des trichterförmigen Aufnahmeelements 6 ist dabei zweckmäßig der typischen Flaschenform von Injektionsmittel-Vorratsflaschen bzw. Spüllösungs-Vorratsflaschen angepasst. Um einen sicheren Halt der Vorratsflasche im Flaschenhalter zu gewährleisten, verfügt die Flaschenaufnahme 2 darüber hinaus über eine oberhalb des Aufnahmeelements 6 angeordnete Halteeinrichtung 7. Die am Aufnahmeelement befestigte Halteeinrichtung 7 weist flexible Halteelemente 8 auf. Die flexiblen Halteelemente 8 umgreifen eine in den Flaschenhalter eingefügte Vorratsflasche an ihrem Außenumfang im zentralen Bereich oder im Bodenbereich der Vorratsflasche, wodurch eine Fixierung der Vorratsflasche in dem Flaschenhalter und insbesondere im Aufnahmeelement 6 gewährleistet wird. Dadurch können Vorratsflaschen von unterschiedlicher Form und Größe sicher in der Flaschenaufnahme fixiert werden.

Bevorzugt sind die flexiblen Halteelemente 8 so ausgebildet, dass sie form- und kraftschlüssig am Außenumfang der Vorratsflasche anliegen. Hierfür weist die Halteeinrichtung 7 wenigstens zwei, an bezüglich der einsetzbaren Vorratsflasche diametral gegenüberliegenden Stellen angeordnete Halteelemente 8a und 8b auf (Figur 1a und Figur 4). Jedes der identisch aufgebauten Halteelemente 8a und 8b umfasst dabei ein paar von ersten Haltelaschen 9a, 9b, welche über ein kreissegmentförmiges Verbindungsteil 9d miteinander verbunden sind. An dem Verbindungsteil 9d ist eine nach unten weisende zweite Haltelasche 9c angeordnet. Die zweite Haltelasche 9c erstreckt sich zwischen den beiden Haltelaschen 9a, 9b des Paars von ersten Haltelaschen und steht in radialer Richtung bezüglich des Innenumfangs des Verbindungsteils 9d vor. Zweckmäßig ist jedes Halteelement 8a, 8b einstückig als KunststoffSpritzgußteil ausgebildet, d.h. das Paar von ersten Haltelaschen 9a, 9b, das Verbindungsteil 9d und die zweite Haltelasche 9c sind einstückig miteinander als Spritzgußteil ausgeformt. Zweckmäßig ist jedes Halteelement 8a, 8b über einen Rast- oder Klemmmechanismus abnehmbar an dem trichterförmigen Aufnahmeelement 6 befestigt. An der Unterseite des trichterförmigen Aufnahmeelements 6 ist ein Spritzschutz 5 vorgesehen, welcher zweckmäßig einstückig mit dem Aufnahmeelement 6 als Kunststoff-Spritzgußteil ausgebildet ist.

Unterhalb der Flaschenaufnahme 2 ist eine Halteeinrichtung 3 zum Anbringen eines austauschbaren Einstechdorns 4 vorgesehen (Figur 2 und 3). In den Schnittdarstellungen der Figur 3 ist ein eingefügter Einstechdorn 4 gezeigt. Die Halteeinrichtung 3 weist eine Führungsschiene oder eine Führungsnut 4 auf. In diese Führungsschiene oder Führungsnut 4 kann ein komplementär ausgeformtes Halteteil eines Einstechdorns 4 eingeschoben werden. Bei dem Einstechdorn 4 handelt es sich zweckmäßig um einen hohlzylindrisch ausgebildeten Dorn mit einer Einstechspitze, an dem das eine Ende eines Zufuhrschlauchs angeschlossen ist, dessen anderes Ende mit dem Verzweigungsstück in Verbindung steht. Alternativ zu dieser Anordnung kann der Einstechdorn auch fest in dem Flaschenhalter integriert sein, indem der Einstechdorn an der Halteeinrichtung 3 befestigt oder mit dieser einstückig ausgebildet ist. Zweckmäßiger ist jedoch die beschriebene Anordnung eines abnehmbar an der Halteeinrichtung 3 befestigten Einstechdorns 4, denn durch diese Ausgestaltung ist eine einfachere und schnellere Reinigung bzw. ein Austausch eines benutzten Einstechdorns gewährleistet.

Zur Herstellung einer Verbindung zwischen dem Zufuhrschlauch und einer in den Flaschenhalter 1 eingeführten und zunächst verschlossenen Vorratsflasche ist ein Mechanismus vorgesehen, mit dem automatisch über den Einstechdorn eine Auslauföffnung in die Vorratsflasche ermöglicht wird. Hierfür ist die Flaschenaufnahme 2 bezüglich des Einstechdorns 4 bzw. bezüglich der Halteeinrichtung 3 für den Einstechdorn beweglich ausgebildet. Bei einer Bewegung der Flaschenaufnahme 2 in vertikaler Richtung auf den Einstechdorn zu wird die in der Flaschenaufnahme 2 befindliche Vorratsflasche so auf den Einstechdorn aufgesetzt, dass der Einstechdorn eine Auslauföffnung in die Vorratsflasche einsticht. Üblicherweise sind solche Vorratsflasche für Kontrastmittel- oder Spüllösungen mit einem leicht von einem Einstechdorn durchstoßbaren Deckel oder einer dünnen Membran verschlossen. Beim Aufsetzen der Vorratsflasche auf den Einstechdom wird daher über den Bewegungsmechanismus des erfindungsgemäßen Flaschenhalters automatisch eine Auslauföffnung in den Deckel bzw. in die Membran der Vorratsflasche eingestochen, wenn die Flaschenaufnahme in vertikaler Grundstellung des Flaschenhalters manuell nach unten auf den Einstechdorn zu geschoben wird. Hierfür muss die Bedienperson lediglich durch Druck auf den Boden der in den Flaschenhalter 1 eingefügten Vorratsflasche diese nach unten drücken, wodurch die Flaschenaufnahme 2 ebenfalls nach unten in Richtung des Einstechdorns geschoben wird.

Der Mechanismus zur Bewegung der Flaschenaufnahme 2 bezüglich des Einstechdorns bzw. der Halteeinrichtung 3 ist aus den Schnittdarstellungen der Fig. 3 zu erkennen. Wie in Fig. 3a gezeigt, umfasst jeder Flaschenhalter 1 ein Gehäuseteil 10, an dem die Halteeinrichtung 3 zum Anbringen eines austauschbaren Einstechdorns 4 befestigt ist. An dem Gehäuseteil 10 ist ein Schwenkhebel 11 befestigt. Der Schwenkhebel 11 ist an einem Schwenklager 12 schwenkbar an dem Gehäuseteil 20 des Injektionskopfs 21 gelagert. Der Mechanismus zum Verschwenken des Flaschenhalters 1 bezüglich des Gehäuseteils 20 von seiner in Fig. 3a gezeigten vertikalen (Grund-)Stellung in eine horizontale Stellung, wird im Folgenden noch erläutert.

An der Unterseite des trichterförmigen Aufnahmeelements 6 ist einstückig mit diesem ein Verbindungsteil 15 angeordnet, welches über eine vertikale Bohrung 36 verfügt. In diese Bohrung 36 ragt eine Führungsstange 16, welche über einen Befestigungsbolzen 35 an dem Verbindungsteil 15 befestigt ist (Fig. 3c). Die Führungsstange 16 weist einen geringeren Durchmesser als die Bohrung 36 auf und erstreckt sich in vertikaler Richtung nach unten bis zum unteren Ende 37 des Verbindungsteils 15. In dem Gehäuseteil 10 ist eine Hülse 38 eingelassen und dort befestigt. Die Hülse 38 weist einen etwas kleineren Durchmesser auf als die Bohrung 36 in dem Befestigungsteil 15 und sie erstreckt sich vom Gehäuseteil 10 in vertikaler Richtung nach oben, bis zum unteren Ende 37 des Verbindungsteils 15. In der Hülse 38 ist eine Druckfeder 12 angeordnet, welche als Schraubenfeder ausgebildet ist und deren unteres Ende auf dem Boden 39 der Hülse 38 aufliegt. Die obere Hälfte der Druckfeder 12 umgreift in ihrem entspannten Zustand (der in Figur 3c gezeigt ist) die Führungsstange 16 und das obere Ende der Druckfeder 12 stützt sich gegen einen Flansch 40 an der Führungsstange 16 ab. Die Flaschenaufnahme 2 ist dadurch in Längsrichtung der Führungsstange 16 und entgegen der Rückstellkraft der Druckfeder 12 verschiebbar. Bei Druck auf die Flaschenaufnahme 2 in vertikaler Richtung nach unten und entgegen der Rückstellkraft der Druckfeder 12, wird die Druckfeder 12 zusammengedrückt und der untere Bereich des Verbindungsteils 15 wird über die Hülse 38 geschoben, wobei die Hülse 38 in die Bohrung 36 eingreift und gleichzeitig die Führungsstange 16 (mit der darum angeordneten Druckfeder 12) in die Hülse 38 eingreift. Durch das Zusammenwirken der Führungsstange 16, des Befestigungsteils 15 und der Hülse 38 wird eine Führungsanordnung gebildet, welche die Bewegung der Flaschenaufnahme 2 in Längsrichtung der Hülse 38 bzw. der Führungsstange 16 führt. Andere Ausgestaltungen dieser Führungsanordnung sind denkbar, so kann beispielsweise die Spiralfeder durch andere Federelemente, wie z.B. Biegefedern oder Blattfedern, ersetzt werden.

Am Befestigungsteil 15 ist weiterhin ein Bolzen 13 mit einer in der Nähe seines unteren Endes angeordneten Nut 14 befestigt (Figur 3a). Der Bolzen 13 erstreckt sich parallel zu dem Führungselement 16 nach unten und greift in eine Bohrung 17 im Gehäuseteil 10 ein. Die Flaschenaufnahme 2 ist längs des Führungselements 16 und entgegen der Rückstellkraft der Druckfeder 12 in der vertikalen Grundstellung des Flaschenhalters 1 zwischen einer oberen (vertikalen) Grenzstellung und einer unteren (vertikalen) Grenzstellung verschiebbar. In Fig. 2 ist die Flaschenaufnahme 2 in ihrer oberen Grenzstellung gezeigt und in der Darstellung der Fig. 3a befindet sich die Flaschenaufnahme 2 in ihrer unteren Grenzstellung.

Wie aus der Schnittzeichnung der Figur 3b zu entnehmen, ist neben dem ersten, durch die Führungsstange 16 gebildeten Führungselement noch ein zweites Führungselement in Form einer zweiten Führungsstange 16a vorgesehen. Diese zweite Führungsstange 16a ist ähnlich wie der Bolzen 13 ausgebildet und am Befestigungsteil 15 befestigt (Figur 3b). Auch die zweite Führungsstange 16a erstreckt sich parallel zu der ersten Führungsstange 16 nach unten und greift in eine Bohrung 17' im Gehäuseteil 10 ein. Am unteren Ende der zweiten Führungsstange 16a ist ein Flansch 34 angeschraubt. Befindet sich die Flaschenaufnahme in ihrer in Figur 3b gezeigten oberen Grenzstellung liegt die Oberseite des Flansches 34 an einer Stufe in der Bohrung 17' an und fixiert auf diese Weise die Flaschenaufnahme 2 in Verbindung mit der Rückstellkraft der Druckfeder 12, welche die Flaschenaufnahme 2 nach oben drückt, in ihrer oberen Grenzstellung, wenn sich der Flaschenhalter in seiner vertikalen Grundstellung befindet. Durch vertikalen Druck von oben entgegen der Rückstellkraft der Druckfeder 12 kann die Flaschenaufnahme 2 von ihrer oberen Grenzstellung in die untere Grenzstellung gedrückt werden.

In der unteren Grenzstellung ist ein Rastmechanismus vorgesehen, über den die Flaschenaufnahme 2 in ihrer unteren Grenzstellung fixierbar ist. Der Rastmechanismus zur Fixierung der Flaschenaufnahme 2 in ihrer unteren Grenzstellung umfasst den Bolzen 13, der die Bohrung 17 durchgreift, sowie eine Rastnase 18, welche in die Nut 14 am unteren Ende des Bolzens 13 eingreift, wenn sich die Flaschenaufnahme 2 in ihrer unteren Grenzstellung befindet. Zur Betätigung des Rastmechanismus ist das untere Ende 19 des Bolzens 13 spitz zulaufend ausgebildet. Wird die Flaschenaufnahme 2 von ihrer oberen Grenzstellung nach unten gedrückt, so bewegt sich der an der Flaschenaufnahme 2 befestigte Bolzen 13 durch die Bohrung 17 des Gehäuseteils 10 und greift in eine am Gehäuseteil 10 befestigte Hülse 26 ein. An der Unterseite der Hülse 26 ist eine federnde Rastnase 18 angeordnet. Beim Durchschieben des spitzen Endes 19 durch die Hülse 20 drückt das spitze Ende 19 des Bolzens 13 die federnde Rastnase 18 zunächst zur Seite weg, bis die Rastnase bei weiterem Einschieben des Bolzens 13 in die Nut 14 eingreift und den Bolzen 13 sowie die daran über das Aufnahmeelement 6 befestigte Flaschenaufnahme 2 in dieser Stellung (untere Grenzstellung) verrastet. Bei der Bewegung der Flaschenaufnahme 2 von ihrer oberen Grenzstellung in die untere Grenzstellung bewegt sich auch die sich in der Flaschenaufnahme 2 befindliche Vorratsflasche auf die Halteeinrichtung 3 und den darin angebrachten Einstechdorn zu, wodurch - wie bereits oben beschrieben - der Einstechdorn eine Auslauföffnung in die Vorratsflasche einsticht. Befindet sich die Flaschenaufnahme 2 in ihrer unteren Grenzstellung, kann - bei dann geöffneter Vorratsflasche - die Pumpe der Injektionsvorrichtung zur Förderung der sich in der Vorratsflasche befindlichen Flüssigkeit in Gang gesetzt werden.

Ist die Vorratsflasche entleert worden, kann der Flaschenhalter 1 über den Schwenkmechanismus von seiner vertikalen Grundstellung in die horizontale Stellung zur Entnahme der geleerten Vorratsflasche geschwenkt werden. Hierfür muss ein Entriegelungshebel 11 (Fig. 1), welcher den Verschwenkmechanismus in seiner Verriegelungsstellung hält, entriegelt werden. Der Entriegelungshebel 11 ist hierfür mit der Schwenkachse 12 gekoppelt, über welche der Schwenkhebel 11 des Flaschenhalters 1 an dem Gehäuseteil 20 des Injektionskopfes 21 angelenkt ist.

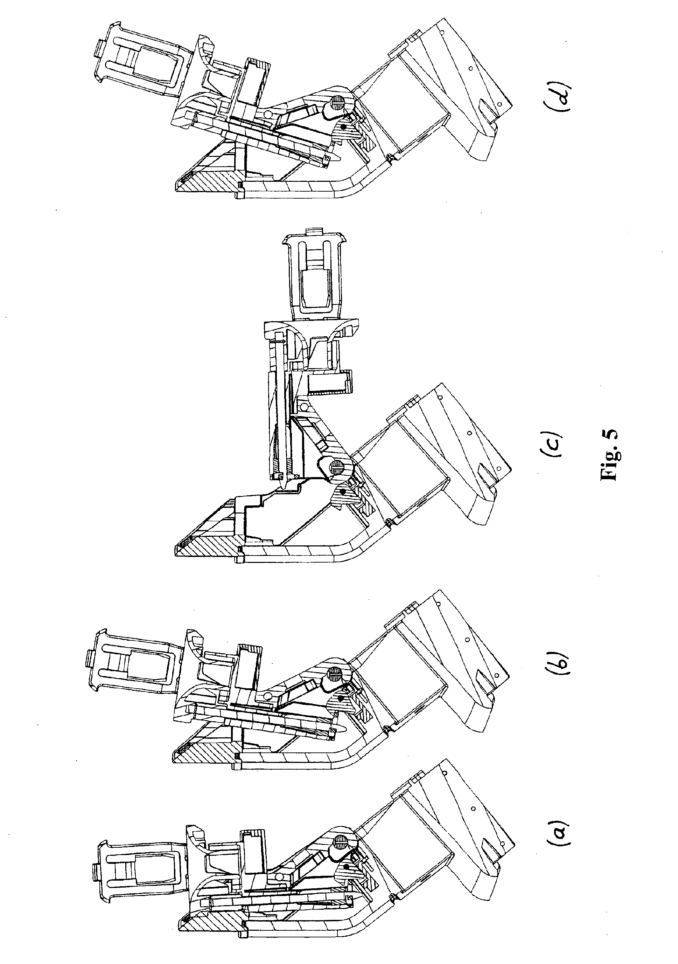

In Fig. 5 sind verschiedene Positionen des Flaschenhalters 1 bezüglich des Gehäuseteils 20 des Injektionskopfes 21 beim Verschwenken des Flaschenhalters 1 von seiner vertikalen Grundstellung (Fig. 5a) über eine Zwischenstellung (Fig. 5b) in seine horizontale Schwenkstellung (Fig. 5c) und wieder zurück in die vertikale Grundstellung gezeigt. Die Darstellung der Fig. 5a entspricht dabei der Darstellung der Fig. 3, in der sich der Flaschenhalter 1 in seiner vertikalen Grundstellung mit der Flaschenaufnahme 2 in der unteren Grenzstellung befindet. In dieser Stellung kann der Flaschenhalter 1 nach Entriegelung des Schwenkmechanismus durch Betätigen des Entriegelungshebels 11 in die in Fig. 5c gezeigte horizontale Stellung verschwenkt werden. Beim Verschwenken des Flaschenhalters 1 von der vertikalen in die horizontale Schwenkstellung verbleibt die Flaschenaufnahme 2 in ihrer unteren Grenzstellung. Dies ist in der Abfolge der Schwenkstellungen in den Fig. 5a, 5b und 5c gezeigt.

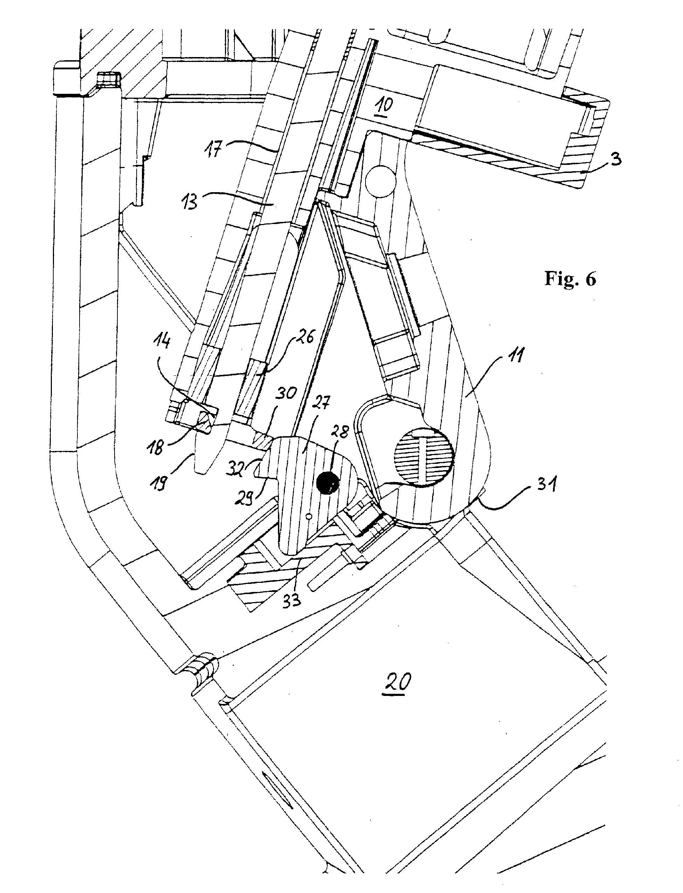

Um zu gewährleisten, dass die Flaschenaufnahme 2 beim Zurückschwenken des Flaschenhalters 1 von seiner horizontalen Schwenkstellung in die vertikale Grundstellung in ihre obere Grenzstellung gebracht wird, ist ein Mechanismus vorgesehen, der die Zurückbewegung der Flaschenaufnahme 2 von ihrer unteren Grenzstellung in die obere Grenzstellung selbsttätig ausführt, während der Flaschenhalter 1 von der horizontalen Schwenkstellung in die vertikale Grundstellung zurückgeschwenkt wird. Dieser in der vergrößerten Ansicht der Figur 6 im Detail gezeigte Mechanismus umfasst einen Verriegelungshaken 27, der an einem Bolzen 28 befestigt ist. Der Bolzen 28 ist in einem am Gehäuseteil 20 befestigten Lagerteil 33 drehbar gelagert (Figur 4). Der Verriegelungshaken 27 weist eine Rastnase 29 auf, welche mit einer an der Hülse 26 angeordneten und mit der Rastnase 18 verbundenen Rastnase 30 zusammenwirkt. In der in Fig. 3 gezeigten unteren Grenzstellung der Flaschenaufnahme 2 liegen die Rastnasen 29 und 30 ohne Eingriff übereinander. Beim Verschwenken des Flaschenhalters 1 in seine horizontale Schwenkstellung kommt die Rastnase 30 mit der Rastnase 29 in Eingriff und zieht den Haken 27 nach oben (Fig. 5b). Dadurch wird der Haken 27 leicht angehoben und durch Drehung des Bolzen 28 gegenüber dem Lagerteil 33 verdreht. Durch die Drehbewegung des Hakens 27 werden die Rastnasen 29 und 30 wieder außer Eingriff gebracht und der Flaschenhalter 1 kann weiter um die Verschwenkwelle 12 verschwenkt werden, bis er seine in Fig. 5c gezeigte horizontale Schwenkstellung erreicht hat. In dieser horizontalen Schwenkstellung liegt die Außenfläche des Schwenklagers 11 an einem Auflager 31 des Gehäuseteils 20 auf und kann nicht mehr weiter nach unten verschwenkt werden. In der horizontalen Verschwenkstellung der Figur 5c kann die geleerte Vorratsflasche tropffrei aus der Flaschenaufnahme 2 herausgezogen werden.

Beim Zurückschwenken des Flaschenhalters 1 von seiner horizontalen Schwenkstellung (Figur 5c) in seine vertikale Grundstellung (Figur 5d) kommt die Rastnase 30 in einer in den Figuren 5d und 6 gezeigten Zwischenstellung zunächst in Anlage an eine Führungsfläche 32 der Rastnase 29. Bei weiterem Zurückschwenken des Flaschenhalters 1 drückt die Rastnase 29 mit ihrer Führungsfläche 32 die Rastnase 30 in Richtung der Schwenkbewegung. Die Rastnase 30 ist mit der flexiblen Rastnase 18 verbunden, wodurch die Rastnase 18 in der Bewegungsrichtung des Rückschwenkvorgangs aus der Nut 14 des Bolzen 13 herausgedrückt wird. Dadurch wird die Verrastung des Bolzens 13 in der unteren Grenzstellung der Flaschenaufnahme 2 freigegeben und die Flaschenaufnahme 2 bewegt sich aufgrund der Rückstellkraft der Druckfeder 12 selbsttätig zurück in ihre obere Grenzstellung. Sobald der Flaschenhalter vollständig in seine vertikale Grundstellung zurück verschwenkt worden ist, hat sich auch die Flaschenaufnahme 2 vollständig in ihre obere Grenzstellung bewegt. Auf diese Weise ist sichergestellt, dass sich die Flaschenaufnahme 2 in ihrer oberen Grenzstellung befindet, wenn der Flaschenhalter 1 in seine vertikale Grundstellung zurück verschwenkt worden ist. In dieser vertikalen Grundstellung kann die Flaschenaufnahme 2 dann mit einer neuen, befüllten Vorratsflasche bestückt werden. Hierzu wird eine neue Vorratsflasche in die Flaschenaufnahme 2 eingeführt und in vertikaler Richtung nach unten gedrückt, um in der oben beschriebenen Weise durch die Bewegung der Flaschenaufnahme 2 in Richtung des Einstechdorns eine Auslauföffnung in die Vorratsflasche einzustechen.

Die Erfindung ist nicht auf das beschriebene Ausführungsbeispiel beschränkt. So kann der erfindungsgemäße Flaschenhalter nicht nur in einer Injektionsvorrichtung zur Injektion von Kontrastmitteln sondern beispielsweise auch in Infusionsgeräten verwendet werden. Anders als bei dem beschriebenen Ausführungsbeispiel können auch weniger als drei Flaschenhalter an einer Injektions- oder Infusionsvorrichtung angeordnet sein. Es können jedoch auch mehr als drei Flaschenhalter der erfindungsgemäßen Art an einer entsprechenden Vorrichtung vorgesehen sein, je nach Anwendungsfall. Es ist auch möglich, einen erfindungsgemäßen Flaschenhalter zur Aufnahme einer Vorratsflasche zusammen mit einem herkömmlichen Beutelhalter zur Aufnahme eines eine zu injizierende Flüssigkeit enthaltenden Beutel zu verwenden.

## Patentansprüche

1. Injektionsvorrichtung, insbesondere zur Injektion von Kontrastmitteln, mit einem Gehäuseteil (20) und wenigstens einem Flaschenhalter (1), welcher eine Flaschenaufnahme (2) zur Aufnahme einer Vorratsflasche und einem am Flaschenhalter (1) befestigten Einstechdorn zum Einstechen einer Auslauföffnung in die Vorratsflasche oder einer Halteeinrichtung (3) zum Anbringen eines austauschbaren Einstechdorns (4) umfasst, wobei die Flaschenaufnahme (2) bezüglich des Einstechdorns (4) oder bezüglich der Halteeinrichtung (3) für den Einstechdorn (4) in vertikaler Richtung zwischen einer oberen Grenzstellung und einer unteren Grenzstellung beweglich ist und bei einer Bewegung der Flaschenaufnahme (2) von ihrer oberen Grenzstellung in ihre untere Grenzstellung eine in der Flaschenaufnahme (2) angeordnete Vorratsflasche so auf den Einstechdorn (4) aufgesetzt wird, dass der Einstechdorn (4) eine Auslauföffnung in der Vorratsflasche einsticht, **dadurch gekennzeichnet, dass** der oder jeder Flaschenhalter (1) verschwenkbar an dem Gehäuseteil (20) der Injektionsvorrichtung angelenkt ist und der oder jeder Flaschenhalter (1) in der unteren Grenzstellung seiner Flaschenaufnahme (2) von einer vertikalen Stellung in eine horizontale Stellung verschwenkbar ist, in welcher eine in der Flaschenaufnahme (2) angeordnete Vorratsflasche eine im Wesentlichen horizontale Lage einnimmt und dass die Flaschenaufnahme (2) beim Zurückschwenken von der horizontalen Stellung in die vertikale Stellung selbsttätig in ihrer oberen Grenzstellung verrastet.

2. Injektionsvorrichtung nach Anspruch 1, **dadurch gekennzeichnet, dass** die Flaschenaufnahme (2) ein trichterförmiges Aufnahmeelement (6) umfasst.

3. Injektionsvorrichtung nach Anspruch 1 oder 2, **dadurch gekennzeichnet, dass** die Flaschenaufnahme (2) eine Halteeinrichtung (7) mit flexiblen Halteelementen (8) umfasst, welche eine in der Flaschenaufnahme (2) angeordnete Vorratsflasche zumindest teilweise an ihrem Außenumfang umgreifen.

4. Injektionsvorrichtung nach Anspruch 3, **dadurch gekennzeichnet, dass** die flexiblen Halteelemente (8) kraftschlüssig und/oder formschlüssig an dem Außenumfang einer in der Flaschenaufnahme (2) angeordneten Vorratsflasche anliegen um die Vorratsflasche in der Flaschenaufnahme (2) zu fixieren.

5. Injektionsvorrichtung nach Anspruch 3 oder 4, **dadurch gekennzeichnet, dass** die Halteeinrichtung (7) wenigstens zwei, an bezüglich der darin einsetzbaren Vorratsflasche diametral gegenüberliegenden Stellen angeordnete Halteelemente (8a, 8b) umfasst.

6. Injektionsvorrichtung nach Anspruch 5, **dadurch gekennzeichnet, dass** das bzw. jedes Halteelement (8; 8a, 8b) ein Paar von ersten Haltelaschen (9a, 9b) und eine daran angeordnete zweite Haltelasche (9c) aufweist.

7. Injektionsvorrichtung nach Anspruch 6, **dadurch gekennzeichnet, dass** das Paar von ersten Haltelaschen (9a, 9b) jeweils über ein kreissegmentförmiges Verbindungsteil (9d) miteinander verbunden sind und dass die zweite Haltelasche (9c) an dem Verbindungsteil (9d) angeordnet ist und sich zwischen den beiden Haltelaschen (9a, 9b) des Paars von ersten Haltelaschen in Längsrichtung der einsetzbaren Vorratsflasche erstreckt.

8. Injektionsvorrichtung nach Anspruch 7, **dadurch gekennzeichnet, dass** die zweite Haltelasche (9c) an dem Verbindungsteil (9d) so angeordnet ist, dass sie in radialer Richtung bezüglich des Innenumfangs des Verbindungsteils (9d) vorsteht.

9. Injektionsvorrichtung nach Anspruch 1, **dadurch gekennzeichnet, dass** die Flaschenaufnahme (2) in der oberen Grenzstellung und/oder in der unteren Grenzstellung über einen Rastmechanismus fixierbar ist.

10. Injektionsvorrichtung nach einem der voranstehenden Ansprüche, **gekennzeichnet durch** ein Gehäuse (10), an dem der Einstechdorn zum Einstechen einer Auslauföffnung in die Vorratsflasche oder die Halteeinrichtung (3) zum Anbringen eines austauschbaren Einstechdorns befestigt ist.

11. Injektionsvorrichtung nach einem der voranstehenden Ansprüche, **dadurch gekennzeichnet, dass** die Flaschenaufnahme (2) bezüglich des Einstechdorns (4) oder bezüglich der Halteeinrichtung (3) für den Einstechdorn (4) gegen die Rückstellkraft einer Druckfeder (12) verschiebbar ist.

12. Injektionsvorrichtung nach Anspruch 10 oder 11, **dadurch gekennzeichnet, dass** die Flaschenaufnahme (2) wenigstens eine Führungsanordnung (15, 16, 38) umfasst und entgegen der Rückstellkraft einer mit der Führungsanordnung (15, 16, 38) gekoppelten Druckfeder (12) verschiebbar ist.

13. Injektionsvorrichtung nach einem der Ansprüche 9 bis 13, **dadurch gekennzeichnet, dass** an der Flaschenaufnahme (2) wenigstens ein Bolzen (13) mit einer Nut (14) angeordnet ist, in welche zur Verrastung der Flaschenaufnahme (2) in ihrer unterer Grenzstellung eine Rastnase (18) eingreift.

14. Injektionsvorrichtung nach einem der Ansprüche 1 bis 13, **dadurch gekennzeichnet, dass** ein Verriegelungsmechanismus vorgesehen ist, welcher den jeweiligen Flaschenhalter (1) in seiner vertikalen Stellung verriegelt und dass der Verriegelungsmechanismus einen Entriegelungshebel (11) umfasst, mit dem die Verriegelung entriegelt werden kann, um den jeweiligen Flaschenhalter (1) von seiner vertikalen Stellung in die horizontale Stellung zu verschwenken.

15. Injektionsvorrichtung nach Anspruch 14, **dadurch gekennzeichnet, dass** der Verriegelungsmechanismus den jeweiligen Flaschenhalter (1) beim Zurückschwenken von der ausgeschwenkten horizontalen Stellung in die vertikale Stellung selbsttätig in seiner vertikalen Stellung fixiert.

## Claims

1. Injection device, in particular for injection of contrast media, with a housing part (20) and at least one bottle holder (1) which comprises a bottle receiver (2) for receiving a supply bottle and a piercing spike fastened to the bottle holder (1) for piercing an outlet opening in the supply bottle or a holding device (3) for attaching an interchangeable piercing spike (4), wherein the bottle receiver (2) is moveable with respect to the piercing spike (4) or with respect to the holding device (3) for the piercing spike (4) in a vertical direction between an upper limit position and a lower limit position and wherein a supply bottle arranged in the bottle receiver (2) is moved onto the piercing spike (4) with a movement of the bottle receiver (2) from its upper limit position to its lower limit position so that the piercing spike (4) pierces an outlet opening in the supply bottle, **characterised in that** the or each bottle holder (1) is articulated pivotally on the housing part (20) of the injection device and the or each bottle holder (1) in the lower limit position of its bottle receiver (2) can be pivoted from a vertical position into a horizontal position in which a supply bottle arranged in the bottle receiver (2) adopts an essentially horizontal attitude and **in that** the bottle receiver (2) is automatically locked in its upper limit position when pivoted back from the horizontal position into the vertical position.

2. Injection device according to claim 1, **characterised in that** the bottle receiver (2) comprises a funnel-shaped receiving element (6).

3. Injection device according to claim 1 or 2, **characterised in that** the bottle receiver (2) comprises a holding device (7) with flexible holding elements (8) which grip around a supply bottle arranged in the bottle receiver (2) at least partially on its external periphery.

4. Injection device according to claim 3, **characterised in that** the flexible holding elements (8) bear in a force-locking and/or form-locking manner on the external periphery of a supply bottle arranged in the bottle receiver (2) in order to immobilise the supply bottle in the bottle receiver (2).

5. Injection device according to claim 3 or 4, **characterised in that** the holding device (7) comprises at least two holding elements (8a, 8b) arranged in diametrically opposing positions with respect to the supply bottle which can be inserted therein.

6. Injection device according to claim 5, **characterised in that** the or each holding element (8; 8a, 8b) exhibits a pair of first holding strips (9a, 9b) and a second holding strip (9c) arranged thereon.

7. Injection device according to claim 6, **characterised in that** the pair of first holding strips (9a, 9b) are each connected to one another by means of a connecting part (9d) with the shape of a segment of a circle and **in that** the second holding strip (9c) is arranged on the connecting part (9d) and extends between the two holding strips (9a, 9b) of the pair of first holding strips in the longitudinal direction of the supply bottle which can be inserted.

8. Injection device according to claim 7, **characterised in that** the second holding strip (9c) is arranged on the connecting part (9d) so that it protrudes in a radial direction with respect to the internal periphery of the connecting part (9d).

9. Injection device according to claim 1, **characterised in that** the bottle receiver (2) can be immobilised in the upper limit position and/or in the lower limit position by means of a detent mechanism.

10. Injection device according to one of the preceding claims, **characterised by** a housing (10) on which the piercing spike for piercing an outlet opening in the supply bottle or the holding device (3) for attaching an interchangeable piercing spike is fastened.

11. Injection device according to one of the preceding claims, **characterised in that** the bottle receiver (2) is displaceable against the restoring force of a compression spring (12) with respect to the piercing spike (4) or with respect to the holding device (3) for the piercing spike (4).

12. Injection device according to claim 10 or 11, **characterised in that** the bottle receiver (2) comprises at least one guide arrangement (15, 16, 38) and is displaceable against the restoring force of a compression spring (12) coupled with the guide arrangement (15, 16, 38).

13. Injection device according to one of claims 9 to 12, **characterised in that** arranged on the bottle receiver (2) there is at least one pin (13) with a groove (14) in which a detent nose (18) engages to lock the bottle receiver (2) in its lower limit position.

14. Injection device according to one of claims 1 to 12, **characterised in that** a locking mechanism is provided which locks the respective bottle holder (1) in its vertical position and **in that** the locking mechanism comprises an unlocking lever (11) with which the lock can be unlocked in order to pivot the respective bottle holder (1) from its vertical position into the horizontal position.

15. Injection device according to claim 14, **characterised in that** the locking mechanism automatically immobilises the respective bottle holder (1) in its vertical position when pivoted back from the extended horizontal position into the vertical position.

## Revendications

1. Dispositif d'injection, en particulier pour l'injection de produits de contraste avec une partie de boîtier (20) et au moins un porte-flacon (1) qui comporte un logement de flacon (2) pour le logement d'un flacon de réserve et une aiguille fixée sur le porte-flacon (1) pour percer une ouverture de sortie dans le flacon de réserve ou un dispositif de retenue (3) pour le montage d'une aiguille (4) remplaçable, le logement de flacon (2) étant mobile par rapport à l'aiguille (4) ou par rapport au dispositif de retenue (3) pour l'aiguille (4) dans le sens vertical entre une position limite supérieure et une position limite inférieure et lors d'un mouvement du logement de flocon (2) de sa position limite supérieure à sa position limite inférieure, un flacon de réserve disposé dans le logement de flacon (2) étant placé sur l'aiguille (4) de sorte que l'aiguille (4) perce une ouverture de sortie dans le flacon de réserve, **caractérisé en ce que** le ou chaque porte-flacon (1) est articulé de manière pivotante sur la partie de boîtier (20) du dispositif d'injection et le ou chaque porte-flacon (1) dans la position limite inférieure de son logement de flacon (2) peut être pivoté d'une position verticale à une position horizontale, dans laquelle un flacon de réserve disposé dans le logement de flacon (2) occupe une position sensiblement horizontale et **en ce que** le logement de flacon (2) s'encliquette automatiquement dans sa position limite supérieure lors du repivotement de la position horizontale à la position verticale.

2. Dispositif d'injection selon la revendication 1, **caractérisé en ce que** le logement de flacon (2) comporte un élément de réception (6) en forme d'entonnoir.

3. Dispositif d'injection selon la revendication 1 ou 2, **caractérisé en ce que** le logement de flacon (2) comporte un dispositif de retenue (7) avec des éléments de retenue flexibles (8) qui entourent un flacon de réserve disposé dans le logement de flacon (2) au moins en partie sur sa périphérie extérieure.

4. Dispositif d'injection selon la revendication 3, **caractérisé en ce que** les éléments de retenue (8) flexibles reposent à force et/ou à complémentarité de formes contre la périphérie extérieure d'un flacon de réserve disposé dans le logement de flacon (2) afin de fixer le flacon de réserve dans le logement de flacon (2).

5. Dispositif d'injection selon la revendication 3 ou 4, **caractérisé en ce que** le dispositif de retenue (7) comporte au moins deux éléments de retenue (8a, 8b) disposés sur des endroits diamétralement opposés par rapport au flacon de réserve insérable dedans.

6. Dispositif d'injection selon la revendication 5, **caractérisé en ce que** le ou chaque élément de retenue (8 ; 8a, 8b) présente une paire de premières languettes de retenue (9a, 9b) et une seconde languette de retenue (9c) disposée dessus.

7. Dispositif d'injection selon la revendication 6, **caractérisé en ce que** les premières languettes de retenue (9a, 9b) de la paire sont reliées entre elles respectivement par une partie de liaison (9d) en forme de segment de cercle et **en ce que** la seconde languette de retenue (9c) est disposée sur la partie de liaison (9d) et s'étend entre les deux languettes de retenue (9a, 9b) de la paire de premières languettes de retenue dans le sens longitudinal du flacon de réserve insérable.

8. Dispositif d'injection selon la revendication 7, **caractérisé en ce que** la seconde languette de retenue (9c) est disposée sur la partie de liaison (9d) de sorte qu'elle dépasse dans le sens radial par rapport à la périphérie intérieure de la partie de liaison (9d).

9. Dispositif d'injection selon la revendication 1, **caractérisé en ce que** le logement de flacon (2) peut être fixé dans la position limite supérieure et/ou dans la position limite inférieure par un mécanisme d'encliquetage.

10. Dispositif d'injection selon l'une quelconque des revendications précédentes, **caractérisé par** un boîtier (10) sur lequel l'aiguille est fixée pour percer une ouverture de sortie dans le flacon de réserve ou le dispositif de retenue (3) pour le montage d'une aiguille remplaçable.

11. Dispositif d'injection selon l'une quelconque des revendications précédentes, **caractérisé en ce que** logement de flacon (2) peut être déplacé par rapport à l'aiguille (4) ou par rapport au dispositif de retenue (3) pour l'aiguille (4) contre la force de rappel d'un ressort de pression (12).

12. Dispositif d'injection selon la revendication 10 ou 11, **caractérisé en ce que** le logement de flacon (2) comporte au moins un ensemble de guidage (15, 16, 38) et peut être déplacé contre la force de rappel d'un ressort de pression (12) couplé à l'ensemble de guidage (15, 16, 38).

13. Dispositif d'injection selon l'une quelconque des revendications 9 à 13, **caractérisé en ce qu'**au moins un boulon (13) avec une rainure (14) est disposé dans le logement de flacon (2), dans laquelle un nez d'encliquetage (18) s'engage pour l'encliquetage du logement de flacon (2) dans sa position limite inférieure.

14. Dispositif d'injection selon l'une quelconque des revendications 1 à 13, **caractérisé en ce qu'**un mécanisme de verrouillage est prévu, lequel verrouille le porte-flacon respectif (1) dans sa position verticale et **en ce que** le mécanisme de verrouillage comporte un levier de déverrouillage (11), avec lequel le verrouillage peut être déverrouillé afin de pivoter le porte-flacon (1) respectif de sa position verticale à la position horizontale.

15. Dispositif d'injection selon la revendication 14, **caractérisé en ce que** le mécanisme de verrouillage fixe le porte-flacon respectif (1) lors du repivotement de la position horizontale pivotée à la position verticale automatiquement dans sa position verticale.
